# EUROPEAN PATENT APPLICATION

(11) **EP 4 020 024 A1**
(43) Date of publication of application: **29.06.2022**
(21) Application number: 20216424.0
(22) Date of filing: 22.12.2020
(51) Int. Cl.: G02B 5/18

(54) **METHOD FOR PRODUCING HIGH ASPECT RATIO FAN-SHAPED OPTICS**

(71) Applicant: Paul Scherrer Institut, 5232 Villigen PSI (CH)
(72) Inventor: STAMPANONI, Marco, 5304 Endingen (CH); SHI, Zhitian, 5413 Kleindöttingen (CH); JEFIMOVS, Konstantins, 5306 Tegerfelden (CH); VILA COMAMALA, Joan, 5408 Ennetbaden (CH); ROMANO, Lucia, 5605 Dottikon (CH)
(74) Representative: Fischer, Michael

(57) **Abstract**

The present invention discloses a method for the fabrication of fan-shaped optical components on a substrate (400), such as those diffraction optics used in X-ray imaging systems. The method uses conductive electric field modulators (301, 302) placed in the peripheral area of the patterned surface on the substrate (400) and plasma etching. The method allows to create an optical component having high aspect ratio elements such as recess structures (401 to 409) with a fan-shape orientation in the substrate (400), wherein each element is inclined by a respective slant angle with respect to the perpendicular to the substrate (400), wherein the said slant angle gradually varies from element to element as a function of the element location on the substrate (400). The method is compatible with conventional plasma etching machines to realize recess structures in the substrate (400) and electroplating techniques to further metallize the slanted recess structures (401 to 409). Fan-shaped gratings in Si substrate with Au filling demonstrated the improved field of view of a grating-based X-ray interferometry set-up, solving among other the problem of bending the optical component in order to efficiently match the cone-beam emission of the light source, such as an X-ray tube.

## Description

The present invention is related to a method to produce optical components with a fan-shaped structure that diffract light wave fronts with an efficient geometry. Further, the present invention is related to the optical components produced by the method.

Optical components, such as optical lenses and diffraction gratings, have long been used to manipulate light for various purposes, such as photonic crystals, LEDs, solar cells and photovoltaic devices. Micro-diffraction gratings are used in the field of light directing devices, such as holographic and augmented/virtual reality, display systems, screens, wearable displays, googles and all that devices where an image is produced by a light source within a certain distance from a detecting device.

The optical components are used to transport, direct and manipulate light. The geometries of an optical component may be designed to achieve various effects and may be different from one region of the device to another region. Optical components, such as optical lenses and diffraction gratings, are usually produced by etching a sequence of recess structures, such as holes or trenches, in a plain substrate. To provide these different regions, different etching methods are used so the geometries of the gratings may differ among the different regions.

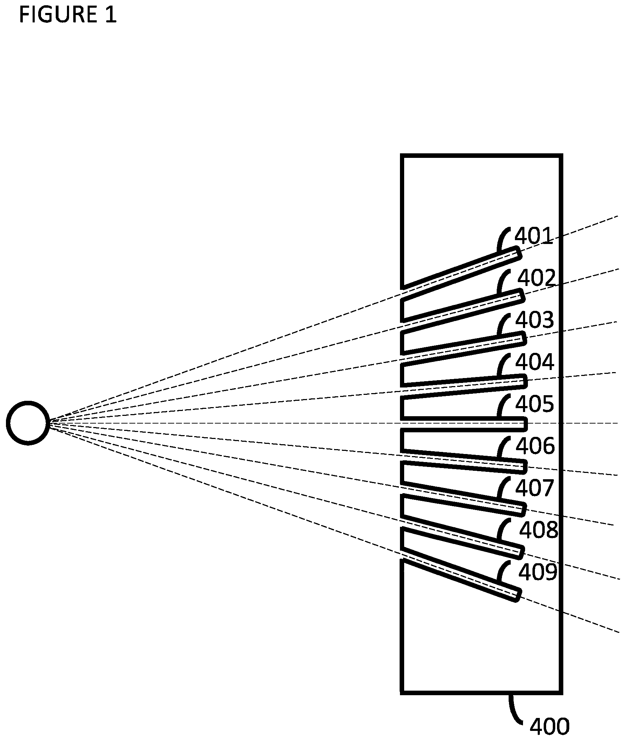

In order to diffract a specific light wave front, the optical component may have a plurality of slanted elements, disposed at non-zero angle of inclination with respect to a direction orthogonal to the plane of the optical component substrate, wherein each slanted element has a different angle with respect to another slanted element. A grating is a periodic sequence of recess structures in a planar substrate. In a fan-shaped grating, the recess structures of the grating elements are slanted at a selected angle with respect to a direction orthogonal to the substrate, each recess structure is slanted by a proper angle with respect to the direction orthogonal to the substrate, wherein the said slant angle increases from one recess structure to the next. An example of 1D fan-shaped grating with multiple slanted recesses is reported in FIG.1.

Each recess lateral surface has a non-zero slant angle with respect to the perpendicular to the substrate. The lateral surface of each recess has a different slant angle with respect to the previous and the following recess, with the slant angle increasing progressively from zero at the center of the device to a non-zero value at the border, with the slant angle increasing symmetrically with respect to the center.

Optical components such as diffraction optics are used in several X-ray imaging methods. X-ray optics are high aspect ratio structures impressed or grown in a planar substrate. X-ray microscopy has been established as a powerful imaging technique and a valuable tool for biological and material sciences. For high resolution scanning and full-field microscopy applications with soft and hard X-rays, Fresnel zone plates provide the highest spatial resolution. Using planar nanofabrication techniques such optics have demonstrated spatial resolutions down to 10 nm. However, this resolution range was only achieved at the cost of low diffraction efficiencies of the used optics. As a consequence, the exposure time and in case of full-field microscopy additionally the radiation load on the specimen is increased. Therefore, the combination of high spatial resolution and high diffraction efficiency still remains an unsolved, fundamental problem in X-ray optics. Based on electrodynamical simulations, the optimized zone plate profile for volume diffraction is given by zone structures with radially increasing tilt angles and decreasing zone heights. On-chip stacking permits the realization of such advanced 3-D profiles without significant loss of the maximum theoretical efficiency. The nanotechnological challenges to fabricate slanted zone structures have not yet been solved.

X-ray free electron laser sources have extended the capabilities of resonant X-ray spectroscopy techniques to ultrafast time scales. This calls for development of new measurement schemes, such as based on off-axis Fresnel zone plates. This approach is based on the fact that the X-rays pulse passing through a different regions of the off-axis optical device arrives to the detector in different time and different location depending on the wavelength. This, on one hand, allows to monitor the spectral properties of the free electron laser pulses themselves. On the other hand, it conveniently allows to observe ultrafast processes on a femtosecond time scale in the samples. The geometric constrains of the experimental setup set the limitation on the optical element design. When the ratio between the focal distance of the zone plate and the radius of the outermost zone become comparable or smaller than the ratio between the height of the outermost zone to its width, the beam diffracted from the outermost zones starts to hit the neighboring lines and fan-shaped geometries with slanted lines become necessary.

X-ray imaging methods have been applied for revealing internal structures of matter, in many application fields like biomedical imaging, non-destructive testing of industrial products, additive manufacturing, geology, archeology, and etc. Grating based X-ray imaging setups essentially detect the deflections of X-rays in an object and can measure the object absorbing, phase shift and dark field signals at the same time. A grating-based X-ray imaging setup usually comprises at least two gratings, an X-ray source, a detector and a recording system. Set-ups with two gratings (G₁ and G₂) or three gratings (G₀, G₁, and G₂) can be applied to record the deflection of the X-rays.

In the case of a two-grating set-up, the X-ray source needs to fulfill certain requirements regarding its spatial coherence, while in a three-grating setup spatial coherence requirements at the source are lessened. Therefore, the three grating set-up is suited for use with incoherent X-ray sources, in particular with X-ray tubes. The gratings are usually placed with gratings lines oriented perpendicular to the central axis of the setup. The grating G₀ is the one closest to the source, G₀ is required before the phase grating G₁ because the X-ray source might not be sufficiently coherent. G₀ consists of a transmission grating of absorbing lines with the period p₀. The grating G₁ is placed further downstream of the X-ray source. G₁ consists of lines with a period p₁. The grating G₂ is the one most downstream of the setup, placed in front of the detector. G₂ consists of a transmission grating of absorbing lines with the period p₂.

Due to the fact that the X-ray source has a cone shape emission, a good alignment of the X-ray beam path is possible only in the center of the imaging set-up, with the misalignment between the X-ray beam path and the grating lines increasing as a function of the distance from the central axis. The misalignment smears the interference fringes, reduces the intensity of the X-ray beam and causes distortions in the phase contrast, increases image noise and limits the effective field of view (FOV).

The X-ray gratings are periodic arrays of lines impressed or grown in planar substrates, the required thickness of the grating lines (i.e. their dimension along the beam path) has to be sufficient to induce sufficient attenuation (in case of absorbing lines) or sufficient phase shift (in case of phase-shifting gratings). Especially for high X-ray energies, for example above 50 keV, the required grating line thicknesses are usually much higher than the period of the grating lines, resulting in very high aspect ratio structures. For medical applications, the X-ray gratings have periods in the range of 0.1 to 100 µm, and aspect ratio higher than 10:1.

The problem of misalignment can be compensated through bending the gratings to a radius equal to the grating distance from the X-ray source. The bending is relatively difficult to accomplish for the G₀ grating, since G₀ is typically placed very close to the X-ray tube within a distance of few centimeters. While the gratings G₁ and G₂ are usually placed at distance in the range from 20 to 100 cm from the X-ray source.

The gratings are usually fabricated by etching silicon substrates and then filling the recess structures with high absorbing material such as Au, or by growing directly the Au gratings in high aspect ratio polymeric templates on flexible substrates such as graphite sheets. In both cases, the grating bendability can be affected by the stress of the grating materials and the flexibility of the substrate. Since the gratings are very high aspect ratio structures, the lines having period in the range of 1 to 10 µm and thickness in the range of 10 to 200 µm, the structures are usually fragile, and the bending procedure can cause distortions and permanent damages. The bending usually requires a permanent deformation of the grating and is not reversible.

The concept of fan-shaped gratings with grating lines aligned with X-ray beam geometry is disclosed with primary axis perpendicular to the substrate surface (see EP 1 731 099 A1 and US 2007/0183583A1), and with primary axis in parallel with the substrate surface (see US 9,036,773 B2). An example of fan-shaped grating aligned with X-ray beam geometry and with primary axis perpendicular to the substrate surface is reported in FIG.1.

Laser beam drilling of a substrate has been proposed as a method to realize fan-shaped grating with primary axis perpendicular to the substrate surface (see EP 2 443 491 B1). However, the laser drilling method has severe limitations in resolution and line roughness, it requires 3D line-to-line alignment and it is intrinsically a slow fabrication process (hours for cm² area), as it requires line by line processing. While high numerical aperture optics allow to focus laser light to sub-micrometer dimensions, the depth of focus of such optics does only allow structuring with low aspect ratios (up to 5:1). Another limitation is the energy deposition to the micromachined surfaces. The high line density of gratings sets a threshold to the energy that sample can absorb without corrugations or cracks of the substrate.

High aspect ratio structures such as the gratings are fabricated by deep reactive ion etching (DRIE) (see US 5 501 893 B2). Like all other plasma etching methods, the etching sections of the Bosch process include physical etching by ion bombardment and chemical etching from reactions with radical etchants where the ion accelerating direction defines the main etching direction. Therefore, the high aspect ratio recess structures produced by DRIE are normally perpendicular to the surface of the substrate.

However, the shape of the electric field that is responsible for the ion acceleration can be influenced by the local topography of the sample surface or by the presence of external structures. Charging effect commonly leads to profile defects such as tilt, notching, bowing and shouldering and these defects worsen with increasing the aspect ratio. A tilt in the etching profile is induced by the potential difference between the etching materials and the charged insulating mask and is dependent on the mask patterns. If a conducting material is used instead of insulating mask, free electrons are able to compensate for the local charging imbalance, and the effect for ion trajectory distortion is limited.

Few modified plasma etching methods can form recess structures with a slant angle with respect to the perpendicular to the substrate:
1) the plasma chamber contains Faraday cages to alter the electric field (see US 9,659,797 B1, WO 2020/004854 A1);
2) inclining and rotating the sample during the etching (see EP 2 136 390 A2, JP 2006/000945A, US 4,309,267 B2, US 2020/004029 A1) ;
3) with insulating protrusions patterned on the substrate (see US 7,226,870 B2); and
4) by introducing a tilt in the etching gas injection direction (see US 2014/295674 A1).

### 1) Faraday cage

Oblique etching is obtained by introducing a Faraday cage to reshape the distribution of the electric field during the etching process. Faraday cages have the advantage of arbitrarily change the shape of the electric field, and the manufacturing is easy and cost effective. However, the sheath layer forms at the surface of the cage and the ions can only be accelerated before they pass through the cage, due to the electromagnetic shielding effect of the Faraday cage itself. Therefore, it is not ideal for deep etching. Besides, the shadow of the metal mesh affects the uniformity of the pattern, and the effect depends on the density of the metal mesh. In general, this method is not the best choice for the fabrication of high aspect ratio gratings, which require high pattern uniformity. Moreover, the Faraday cages are placed right above the area to be etched, with high risk of contamination by metal particles during the etching process.

### 2) Inclined substrate

Oblique etching is obtained by inclining and rotating the sample stage at certain tilts during the etching process, in order to modify the direction between the accelerated ions and the surface of the sample. However, only uniformly tilted etching with a single angle all over the sample can be created. Therefore, the method of rotating the substrate is not appropriate for the fabrication of deep recess structures with multiple slant angles such as the fan-shaped structures.

### 3) Injecting ions with a tilt

Ions injection is usually performed with the ions' trajectories perpendicular to the substrate surface. Oblique etching can be performed by injecting the etchant gases with a non-zero angle with respect to the perpendicular of the substrate. Similarly, to the method of rotating substrate, injecting ions with a tilt can form only uniformly tilted recess structures, without multiple slant angles.

### 4) Insulating protrusions

The surface of the photoresist collects more electrons than the substrate during plasma etching process, therefore a weakly deformed electric field could be formed. The trajectory is then slightly altered before the ions arrive at the surface of the substrate. This method is not suitable for deep etching, since only very limited regions can be affected.

Thus, so far, no feasible methods have been reported to fulfill the requirements of fan-shaped optics fabrication described above, with high aspect ratio recess structures (higher than 10:1), with a plurality of slanted elements with respect to the perpendicular to the substrate.

These requirements are achieved according to the present invention by a method for fabricating an optical component having high aspect ratio recess structures with a fan-shape orientation in a substrate, wherein each recess structure is inclined in either one or more freely selectable directions in planes perpendicular to the surface of the substrate, wherein the recess structure direction forms a slant angle with the perpendicular to the surface of the substrate, wherein the said slant angle gradually varies from one recess structure to the next recess structure as a function of the location of the recess structure on the substrate, comprising the steps of:
a) providing a substrate having a pattern of the optical component on the upper surface, wherein said pattern serves as a mask for a following etching step;
b) placing one or more electric field modulators next to the patterned surface of the substrate;
c) inserting the patterned substrate and the electric field modulators into a chamber of a plasma etching system, wherein the electric field modulators are insulated from an etcher cathode in the etching chamber;
d) forming the high aspect ratio recess structures in the patterned substrate by means of etching with accelerated ions in the modified electric field formed by the presence of the electric field modulators located next to the patterned surface of the substrate.

Thus, the present invention provides a method to realize slanted elements in a planar substrate by using ion etching plasma systems of a patterned substrate and by locally modifying the electric field on the surface of the substrate in a plasma etching system by means of the electric field modulators. Preferred embodiments of the present invention are given in the attached dependent claims 2 to 14.

With respect to a preferred use of the optical component, the present invention also provides an apparatus for grating based X-ray imaging, comprising:
a) an X-ray source;
b) an X-ray sensitive detector;
c) a set of three gratings positioned between the X-ray source and the detector;
d) at least one of the gratings having fan-shape structure in a substrate and being manufactured as described above;
e) at least one of the said gratings having fan-shape structure has the volume of the etched recess structures at least partially filled with a material selected from the group consisting of Au, Ir, Pt, Ru, Os, W, Gd, Ta, Pb, Bi, Sn, Cu, Ni and combination thereof;
f) at least one of the filled fan-shaped grating is positioned between the X-ray source and one of the gratings acting as phase grating; and
g) the maximum slant angle of the said fan-shaped grating is 45°.

Further preferred embodiments are listed in the remaining dependent claims.

Preferred embodiments of the present invention are hereinafter described in more detail with reference to the attached drawings which depict in:
- FIG 1: a schematic sectional view of a 1D fan-shaped grating according to the prior art;
- FIG 2: schematically an arrangement of substrate, sample chip, bonding materials, and electric field modulators according to the embodiments of the present disclosure with the electric field modulators placed next to the pattern where the slanted recess structures are etched in the substrate;
- FIG 3: schematically an arrangement of substrate, sample chip, bonding materials, and electric field modulators according to the embodiments of the present disclosure with the electric field modulators placed or coated on top of to the pattern where the slanted recess structures are etched in the substrate;
- FIG 4: schematically different examples of substrate metal filling: A) Filling of the structure with or without removing the metal etching mask; B) filling of the structure through the top layer of the double layer substrate, when the bottom layer acts as a seeding layer for electroplating;
- FIG 5: schematically the simulated electric field under the influence of modulators with cross section shape of A) rectangle; B) quarter circle; C) triangle, wherein the bias power is set at -200 V; arbitrary units on both axes;
- FIG 6: schematically some examples of 2D fan-shaped recess structures: A) frustums; B) units with concentric circles; C) pillars; D) Fresnel zone-plate; E) linear Fresnel optics;
- FIG 7: scanning electron microscopy images in cross section of etched 1D fan-shaped grating with electric field modulation during the deep reactive ion etching process according to the embodiments of the present disclosure, wherein the slant angle is measured in each image as a function of the position;
- FIG 8: schematically a typical three grating X-ray interferometry setup; system with conventional G₀ grating: schematic of set-up (A), the transmission image (B) and differential phase contrast image of a test sample; a system with a 1D fan-shaped G₀ₛ grating: schematic of set-up (D), the transmission image (E) and differential phase contrast image of a test sample (F);

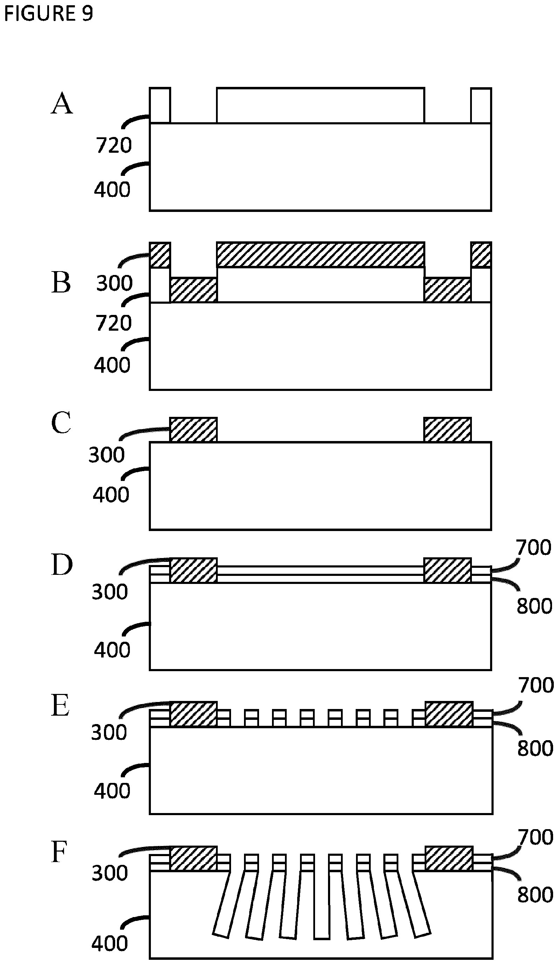

- FIG 9: schematically an example of a fabrication flow of fanshaped recess structure, where the modulators and the fan-shaped structure are produced utilizing two separate lithography steps: A) first lithography step which defines the dimensions of the modulators in the photoresist layer; B) deposition of the modulator material; C) stripping of the photoresist layer; D) deposition of the hard mask and photoresist layer for a second lithography step; E) second lithography step, which defines the pattern of the optical device in photoresist layer, and transfer to an optional hard mask layer; F) etching step of fan-shaped recess structures;
- FIG 10: the schematic of slant angle distribution in the fanshaped recess structure as a function of the distance from the modulator in case of: A) two symmetric modulators (vertical structure in the center) and B) one single modulator (off-axis fan-shape).
- FIG 11: schematically the simulated electric field under the influence of modulators in case of: A) one single modulator; B) two modulators with asymmetric size; C) two modulators with asymmetric shape; the bias power being set at -200 V.
- FIG 12: an example of an 2D fan-shaped recess structure produced in silicon: A) schematic of etching arrangement (carrier wafer, patterned chip and electric field modulator for 2D fan-shaped structure; B) scanning electron microscopy images in plan view of etched 2D fan-shaped array (chessboard grating) with electric field modulation during the deep reactive ion etching process according to the embodiments of the present disclosure.

In X-ray optics, strong distortions may arise in regions outside the center of the field of view due to the cone beam emission of an X-ray source such as a laboratory X-ray tube. The issue can be solved by using an optical component with a plurality of elements with different inclination (slant angle) with respect to a central axis (fan-shape) and aligning each element with a different beam direction of the cone beam. The method of the present disclosure solves the problem to fabricate diffractive optics with a fan-shaped structure by etching features in a substrate with high pattern uniformity and high aspect ratio, such as gratings for X-ray imaging and X-ray interferometry systems.

The method of the present disclosure reduces the processing steps and cost with respect to prior art. It is cost effective since it does not require a complex tool modification such as in the case of changing the ion flux direction or tilting the substrate during etching or producing the slant angle by a specific lithographic mask. The method of the present disclosure allows higher pattern uniformity, higher aspect ratio and lower metal contamination with respect of using Faraday cage to shape the distribution of the electric field during the etching process. With the method of this invention, the gratings can be easily and cost effectively prepared with a predesigned geometry in 3D that matches the beam divergence of the X-ray source, without the need of a mechanical bending of the device.

The method is compatible with regular RIE machines. Linear gratings with fan-shaped structure in silicon, polymer or other dielectric or semiconductor substrates, realized with the disclosed method can be metallized by common electroplating techniques, such as electroplating, atomic layer deposition, casting, and etc. The method can be used to produce more complex fan-shaped optics in 3D, such as Fresnel zone-plates, arrays of circular gratings, chessboard gratings, arrays of slanted pillars etc. Fan-shaped structure can be produced by the disclosed method in symmetric or non-symmetric distribution of the slant angle with respect to the center of the patterned device.

In detail, the fan-shape structure includes elements separated by high aspect ratio recess structures, wherein each element is inclined in either one or more directions in planes perpendicular to the surface of the substrate, wherein the element direction forms a slant angle with the perpendicular to the surface of the substrate, wherein the said slant angle gradually varies from element to element as a function of the element location on the substrate. The present invention provides a method to realize slanted elements in a planar substrate by using ion etching plasma systems of a patterned substrate and by locally modifying the electric field on the surface of the substrate in a plasma etching system by means of metal modulators. The metal modulators are metallic slabs with several cross section, such as squares, triangles and semicircles, providing sufficient local electric field modulation in the sheath of the plasma so to slant the angle of the impinging ions and to create a gradual and uniform variation of slant angle in the etched recess structure. Thus, the present invention relates to the fabrication of a big variety of slanted elements, 1D and 2D fan-shaped structures, as a function of the preliminary formed pattern on the surface of the substrate. The pattern is realized by means of conventional lithography and eventually transferred on hard masks on the substrate. The slanted elements are separated by high aspect ratio slanted recess structures that are eventually filled up with a material different from the substrate.

More specifically, the invention provides a method for forming high aspect ratio 1D fan-shaped grating in a silicon substrate. After filling this grating with an absorbing material, the invention relates to the use of the 1D fan-shaped grating in a grating-based X-ray imaging set up after the X-ray tube and before a phase grating in order to compensate the effect of cone beam geometry on the borders of the field of view.

To clarify, in the drawings the same reference numerals apply for the same elements throughout the entire discussion herein. The drawings are very schematic and are not to scale.

FIG 1 schematically illustrates the fan-shaped grating realized in one embodiment of this disclosure. The substrate lamella between two subsequent recess structures 401 to 409 constitutes a grating line. In case of small variation of the angle among the two surfaces belonging to the same recess structure, each grating line has approximately one main slant angle. The axis of symmetry is located in the center of the grating and is called the primary axis (passes through recess structure 405). The angles of all the grating lines are referred to the primary axis. The grating line 405 that coincides with the primary axis is perpendicular to the surface of the substrate 400. The slant angle of the grating line increases gradually as a function of the distance from the primary axis.

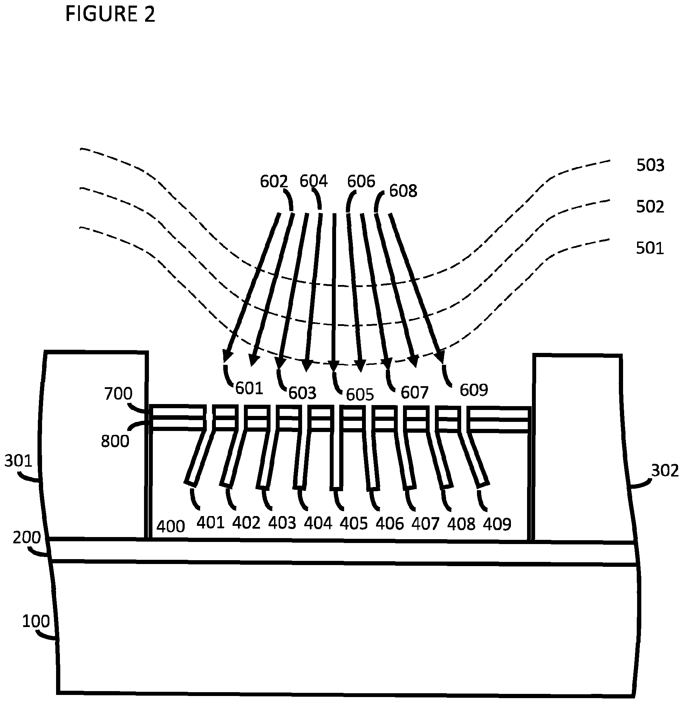

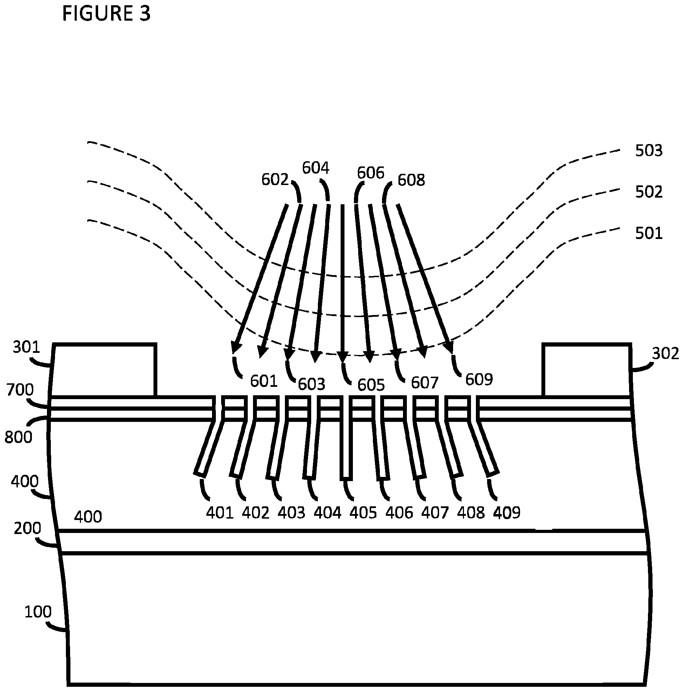

The process flow for fabricating the fan-shaped gratings according to the embodiments of this disclosure is described in the following. The substrate 400 with designed grating pattern is prepared, then the substrate could be either first trimmed into desired shape or first patterned with grating lines. Sample trimming is performed by cleaving, or laser cutting, or saw dicing, or etching. The grating pattern is prepared by lithographic methods. The lithographic method to realize the grating pattern includes but is not limited to: UV mask lithography, displacement Talbot lithography (DTL), electron-beam lithography, imprinting lithography. Hard masks 800 (see Fig. 3) may or may not be introduced depending on the target height of the grating lines. In some embodiments of this disclosure, electric field modulators 301, 302 (see Fig. 2) are made out of highly conductive materials, typically metals, including but not limited to: Al, Au, W, Ag, Pt, Cu, Fe, Ni, steel, graphite, and combination thereof.

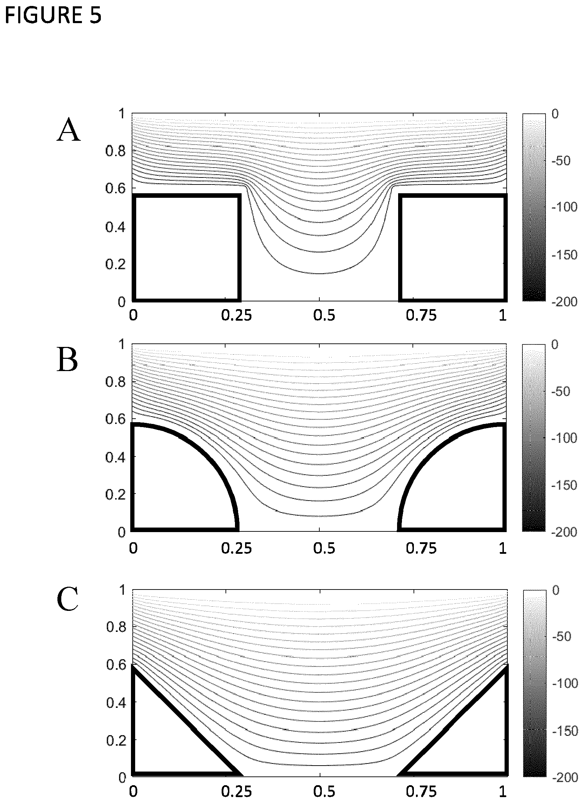

In some embodiments of this disclosure, the electric field modulators 301, 302 are metal bars insulated from the carrier wafer. This allows the accumulation of electrons during plasma etching process. The electrons will stay at the surface of the metal due to a skin effect, and the high electron mobility of metals ensures a uniform electric field. The parts numbered as 301, 302 are electric field modulators. The size of the modulators section is related to the design parameters of the grating, such as the slant angle and the grating area. As a general trend, thicker electric field modulators deliver a larger maximum slant angle and are used for the fabrication of patterns with large area. While, wider electric field modulators enable the fabrication of large area gratings with uniform angle distribution. The width of the electric field modulators is in the range of half the size of the whole fan-shaped grating pattern. The shape of the modulators is related to the grating pattern design. For producing a 1D fan-shaped linear grating the modulators are long bars with section shape of rectangle. The shape of the modulators section includes but is not limited to: square, rectangle, triangle, trapezoid, semicircular. Some examples of corresponding electric fields under the influence of such modulators are simulated in FIG 5 with a bias voltage of - 200 V, horizontal and vertical scales have arbitrary units, grey scale is in V.

As shown in Fig. 2, the substrate 400 and the electric field modulators 301, 302 are attached to a carrier wafer 100 with adhesive mediums 200, such as bonding waxes, epoxy resins or polyimide films or tapes. In case of dielectric substrate or a substrate coated with insulated layer the metal modulators can be placed to the carrier substrate without adhesive media in between. The entire structure composed of the substrate 400, the electric field modulators 301, 302, together with the carrier wafer 100, is inserted into a plasma etching chamber. The grating lines 401 to 409 are etched into the substrate 400 by a plasma etching method. The plasma etching method includes but is not limited to: deep reactive ion etching (DRIE), Bosch etching, cryogenic reactive ion etching, inductively coupled plasma, radiofrequency plasma etching, ion beam assisted etching, ion milling, scanning focused ion beams, ion implantation, atomic layer etching. The substrate 400 may or may not be composed of a multiplicity of layers, each layer includes but is not limited to: Si, SiO₂, Al₂O₃, Ge, Si-Ge alloys, SiN, SiC, diamond, graphite, Mo, W, C, Ta, glass, polymers, and combination thereof.

In some embodiments of the present disclosure, the substrate 400 is a silicon wafer and two metal slabs are used as electric field modulators 301, 302. The metal slabs are placed next to each side of the substrate 400, with long edges in parallel with the grating lines as shown in FIG 2.

FIG 2 also illustrates the local electric field in the proximity of the substrate 400 during the etching process. Dotted lines 501 to 503 schematically represent the equipotential lines of the electric field produced by the electric field modulators. The arrows 601 to 609 schematically represent the accelerating direction of the ions under the influence of the local electric field; the ions are directed perpendicular to the equipotential lines. Since most of the kinetic energy of the ions is acquired from acceleration in the electric field, the angle of the etched grating line aligns with the ion incoming direction. In presence of electric field modulators 301, 302 made out of insulating materials, the electrons produced in the plasma accumulate at the modulator surface. In presence of electric field modulators 301, 302 made of conductive materials and insulated from the ground due to the presence of an insulating layer, the electrons are mainly adsorbed and dispersed in the modulators' bodies, a minor part of the adsorbed electrons are accumulated at the surface of the modulators under the influence of positive potential in plasma body and Coulomb force.

In both cases of conductive and insulating modulators, the modulators alter the electric field in the proximity of the substrate 400. In presence of electric field modulators 301, 302 made of insulating material, the morphological profile of the electric field modulator 301, 302 produces remarkable noise in the modulation of the electric field in the proximity of the surface of the substrate 400. The noise in the modulation of the electric field due to the roughness of the insulating modulator surface can remarkably affect the uniformity of the etched elements in the substrate 400. Since the electrons can travel freely inside and at the surface of the metallic modulators, the morphological profile of the modulator and the surface roughness of the metal are smoothed out in the modulated electric field in the proximity of the substrate surface. The conductive modulator is intrinsically different from a Faraday cage since the modulator only exists in the peripheral area around the pattern, while the Faraday cage is right above the area to be etched. Therefore, the contamination of metal particles can be avoided with modulators, and the electric field modulated by the metallic modulators results in a uniform distribution and a smooth gradient of slant angles.

In some embodiments of this disclosure, the electric field modulators are made of conductive materials and the modulators are isolated from the ground by insulating adhesive medium or additional insulating layer between the modulator and the etcher cathode. In some embodiments of this disclosure, fan-shaped optics are fabricated with slanted elements, wherein the slant angle varies smoothly from element to element with a uniform and continuous gradient due to a smooth modulation of the electric field. Moreover, optical components and lenses are usually geometrically symmetric with respect to the optical axis.

Electric field modulators 301, 302 can be produced with equal shape and equal geometrical features (see FIG. 5) in order to be used to fabricate symmetric optical devices. Electric field modulators 301, 302 can be made of bulk materials by means of but not limited to sawing, water jet, laser ablation or laser cutting. Electric field modulators 301, 302 can be made by means of but not limited to additive manufacturing, 3D printing and casting of conductive materials. The unevenness in the surface features of the electric field modulators 301, 302 can be very difficult to smooth out by means of grinding or polishing, especially if the electric field modulators 301, 302 are made of soft material, such as polymer. The free travelling of electrons in the metallic modulators enables the electric field to be symmetric even in presence of unevenness in the surface of the modulators.

A symmetrically modulated electric field (see FIG. 5) produces a symmetric distribution of slant angles in the etched elements with respect to the perpendicular to the substrate, which is a preferred condition for the fabrication of on-axis optical components and lenses, being these devices symmetric with respect to the optical axis.

In some embodiments of this disclosure, the electric field modulators 301, 302 are placed on top of the surface of the substrate 400. The electric field modulators are metallic elements composed by a plurality of layers including but not limited to: Al, Au, W, Ag, Pt, Cu, Fe.

An example of electric field modulators 301, 302 laying on the surface of the substrate 400 is reported in FIG 3. In some embodiments of this disclosure, the metallic modulators are realized by foils of metals with opened areas, wherein the opened areas define the regions of the substrate to be patterned. The said opened areas in the metal foils are realized by means of a lithographic method followed by an etching method or directly by laser cutting or diamond saw. In some embodiments of this disclosure, the metallic modulators are realized by means of a lithographic method, the metal pattern of the metallic modulators may or may not lay on top of a polymeric layer 700, wherein the polymeric layer 700 includes but is not limited to photoresists, imprint resist, electron or ion beam resist. The metal layer is realized by means of but is not limited to: evaporation, sputtering, electrodeposition, laser ablation, electroplating, electro-less plating.
In some embodiments of this disclosure the substrate 400 is selected from the group of materials consisting, but not limited to: Si, SiO₂, Al₂O₃, Ge, an alloy of Si and Ge, SiN, SiC, diamond, graphite, Mo, W, C, Ta, glass, polymer, elements and alloys from the groups III and V of the periodic table, elements and alloys from the groups II and VI of the periodic table, and combination thereof.

In some embodiments of this disclosure the substrate 400 is a multilayer structure, wherein any layer is composed from the group of materials consisting, but not limited to: Si, SiO₂, Al₂O₃, Ge, an alloy of Si and Ge, SiN, SiC, diamond, graphite, Mo, W, C, Ta, glass, polymer, elements and alloys from the groups III and V of the periodic table, elements and alloys from the groups II and VI of the periodic table, and combination thereof.

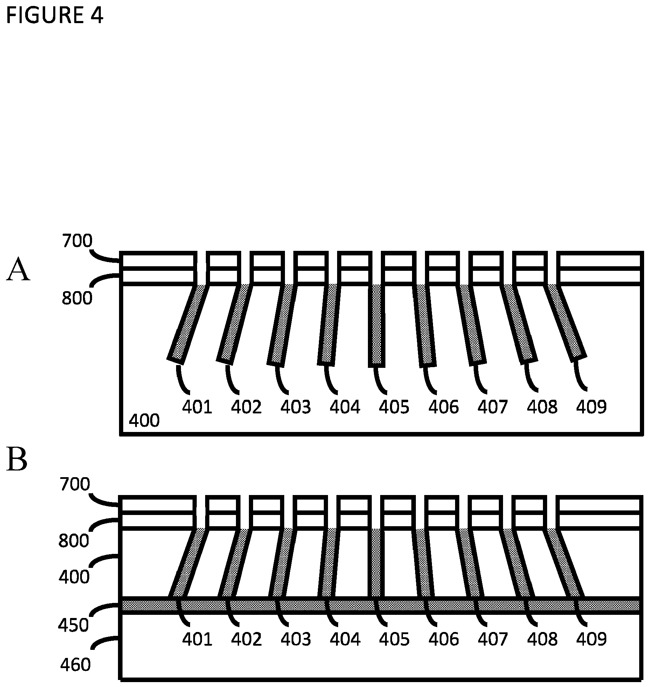

The high aspect ratios are etched into top layer of the substrate 400 and a conductive bottom layer 450 is served as an etching stop layer. In some embodiments of this disclosure, the conductive bottom layer 450 is served as conductive electroplating base for filling the high aspect ratio recess structures by electroplating. In some embodiments of this disclosure, the substrate is a multilayer structure, consisting of three or more layers (see FIG. 4B) whereas a top layer of the substrate 400 is a dielectric, a bottom layer 460 can be composed of any material and at least one of the stacks of intermediate layers comprises the conductive material layer 450. Whereas, in some embodiments, the intermediate layers contain adhesion layers. Whereas, in some embodiments, the intermediate layers contain etch stop layers.

In some embodiments of this disclosure, the substrate 400 is a multilayer structure, consisting of two layers (see Figure 4B) whereas the top layer of the substrate 400 is selected from the group of materials consisting, but not limited to: Si, SiO₂, Al₂O₃, Ge, an alloy of Si and Ge, SiN, SiC, diamond, graphite, Mo, W, C, Ta, glass, polymer, elements and alloys from the groups III and V of the periodic table, elements and alloys from the groups II and VI of the periodic table, and combination thereof and the bottom layer 450 is conductive material selected from the group of materials consisting, but not limited to: Ti, Cr, Ta, W, Cu, Bi, Au, Pt, Ge, Si, Ni, and combination thereof. The layer 460 may or may not be absent.

In some embodiments of this disclosure, the metal modulators and the fan shaped structure (see FIG. 3) are produced utilizing two separate lithography steps (FIG. 9).

In one embodiment, the fabrication flow includes the steps, as described below. A deposition of photoresist with a thickness 0.5-10 µm and a first photolithography step, which defines the dimensions of the modulator in the photoresist layer 720, is performed (FIG. 9A). The modulator pattern is a surrounding structure, such as for example a ring, with inner dimensions in a range of 0.1-20 mm and a width in a range of 0.1-10 mm. The electric field modulator 300 is deposited utilizing thermal evaporation process, whereas the material of the electrical field modulator 300 is a metal, such as for example Al or Ni, whereas the thickness of the modulator 300 is in the range 0.1-100 µm. A lift-off process is performed to strip the residuals of the photoresist layer 720 and the metal layer deposited on top of the photoresist 720 with only the pattern of the electric field modulator 300 remaining on the substrate 400 (FIG. 9C). Optionally, a hard mask 800 and a further photoresist layer 700 for a second lithography step are deposited (FIG 9D). A second lithography step, which defines the pattern of the optical device in the photoresist layer 700, is performed at the center of the modulator and transferred to an optional hard mask layer 800 (FIG. 9E). Finally, the etching step (FIG. 9F) results in the structure shown in FIG. 3.

In some embodiments of this disclosure, electric field modulators are alternatively produced by Pt deposition in a focused ion beam system from a metallic precursor. The advantage of this method is the reduction of lithographic steps and the precision of pattern definition for small structures in the range of few millimeters.

In some embodiments of this disclosure, the substrate 400 shown in Figures 2 and 3 is etched through. The recess structures 401 to 409 of the as-prepared fan-shaped structure following the process described above may or may not be filled with other materials that are different from the material of the substrate 400 (FIG. 4A). The filling material may partially or completely fill up the volume of the substrate recess structures 401 to 409. The filling material partially or completely filling the substrate recess structures may be but is not limited to a continuous layer of material different from the substrate or particles of material different from the substrate. The filling material partially or completely filling the substrate recess structures may be deposited within the substrate recess structures by means of but is not limited to: evaporation, laser ablation, chemical vapor deposition, atomic layer deposition, sputtering, epitaxy, electroplating, electro-less plating, crystal growth from a seed layer, casting from melted alloys, casting from solutions of dispersed particles, powder compression, particles precipitation from solvent evaporation, sol-gel synthesis or a combination of the above filling methods.

Depending on the configuration of the grating-based X-ray set-up and functionality of the specific grating, the grating can be used as phase shifting or absorbing grating. In some embodiments of the present disclosure, the volume of the slanted recess structures 401 to 409 in a Si substrate is completely filled with Au by means of Au electroplating and used as absorption grating in a grating-based X-ray imaging set up. In some embodiments of the present disclosure, the volume of the slanted recess structures in the substrate is partially filled with Ir by means of atomic layer deposition and used as absorption grating in a grating-based X-ray imaging set up. In general, absorption gratings have the grating recess structures 401 to 409 partially or completely filled with materials that have higher X-ray absorption coefficient than the substrate 400. In some embodiments of the present disclosure, partially or completely filled gratings are used as phase shifting gratings. The slanted recess structures 401 to 409 can also be filled with a material different from the substrate with higher absorption efficiency of X-rays.

In the present disclosure, the filling of the slanted recess structures 401 to 409 is realized with at least one of the high absorption materials, including but not limited to: Au, Ir, Pt, Ru, Os, W, Gd, Ta, Pb, Bi, Sn, Cu, Ni and a combination thereof. The slanted recess structures 401 to 409 in the substrate 400 can also be filled with a material different from the substrate 400 with scintillator properties. In some embodiments of the present disclosure, the volume of the slanted recess structures 401 to 409 is partially or completely filled with a scintillator material. Some examples of scintillator materials are selected from but not limited to: Gd, Gd₃Ga₅O₁₂:Tb, CdWO₄, and Y₃Al₅O₁₂:Ce. The gratings with recess structures filled up with scintillator materials are used to improve the detection efficiency and spatial resolution in detectors systems. When an exciting radiation impinges on the gratings filled up with the scintillating material, only those areas with scintillating material emit visible light, and the visible lights is recorded directly by photodetectors. The exciting radiation may be but is not limited to photons with energy of X-rays.

In some embodiments of the present disclosure, the fan-shaped recess structures are made in heavily doped Si substrates and are used as optics for neutrons, wherein the dopant elements of the used Si substrates have high neutron attenuation properties. Fan-shaped gratings in doped Silicon substrates with elements of high neutron attenuation can be used for interferometry with neutrons. Elements with high neutron attenuation properties are selected but not limited to: Gd, B, Cd, Co, Hf, Ti, Mo or combination thereof.

In some embodiments of the present disclosure, the volume of the fan-shaped recess structures in a Si substrate is partially or completely filled with material containing elements with high neutron attenuation, such as Gd, B, Cd, Co, Hf, Ti, Mo or combination thereof, and used as grating for interferometry with neutrons.

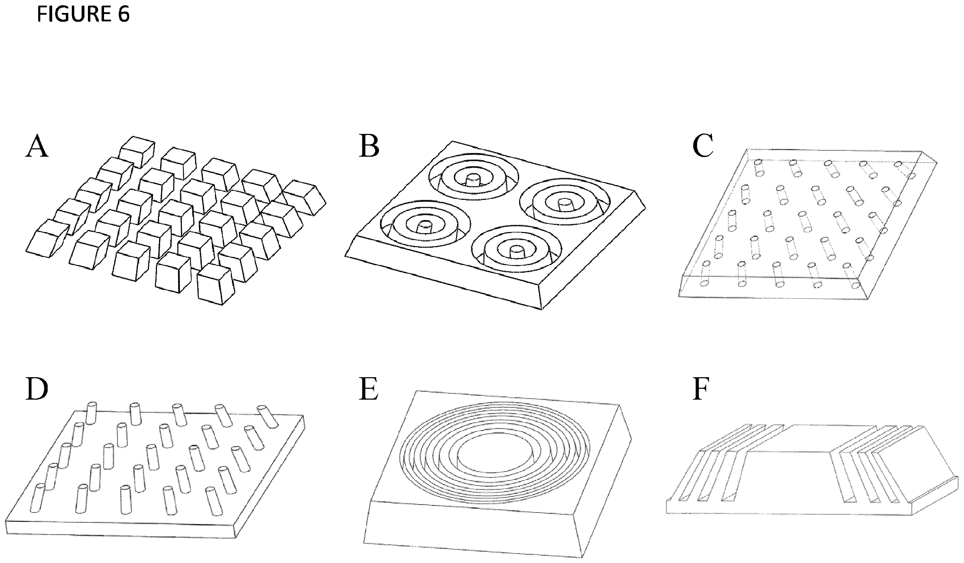

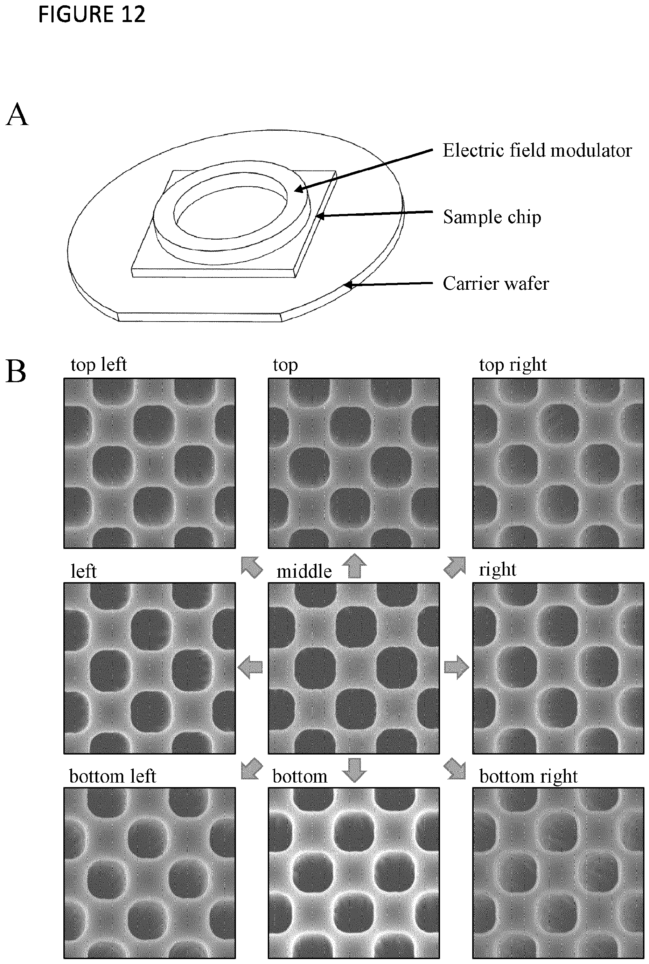

In some embodiments of the present disclosure, the electric field is modulated in two dimensions in order to fabricate a complex optical component with a plurality of slanted elements such like the ones reported in FIG 6. FIG 6 shows some complex slanted recess structure arrays with high aspect ratios, which are called 2D-fan shaped structures. The electric field is modulated in two dimensions by applying electric field modulators to the entire periphery of the substrate. The pattern of the substrate includes but is not limited to: array of slanted pillars with rectangular or squared cross section (FIG 6A); array of slanted concentrically circular omnidirectional gratings (FIG 6B); array of slanted holes (FIG 6C); array of slanted pillars with circular cross sections (FIG 6D); array of slanted pillars with rectangular or squared cross section and a chessboard arrangement; slanted Fresnel zone plates (FIG 6E) wherein the circular grating has the period decreasing in size from center to the edge; slanted linear Fresnel zone (FIG 6F). The patterns reported in FIGURE 6 are representative examples of slated optics and the disclosed method is not limited to the patterns described in FIG.6. The number of unit cells in the array patterns is not limited to the examples slowed in FIG.6. Any combination of patterns of FIG.6 in arrays can be realized within the disclosed method. For example, a unit cell with a slanted Fresnel zone plates (FIG 6E) can be combined in an array similar to FIG.6B, in order to provide a set of omnidirectional gratings with additional diffractive properties. Other arrangement of 2D patterns are possible, such as chessboard gratings (FIG 12B). The method of this disclosure allows the fabrication of any geometrical pattern in two dimensions on a substrate, wherein the pattern involves the formation of a plurality of recess structures in the third dimension of the substrate, wherein the said plurality of recess structures leads to the formation of 3D elements with high aspect ratio in the substrate, wherein the said 3D elements have directions in either one or more perpendicular plane to the substrate surface and forming a plurality of slant angles with respect to the perpendicular of the substrate surface.

In reference to FIG 6, each element arising for the etching of a set of recess structures can individuate a slant angle in one or more than one perpendicular plane to the substrate. In reference to FIG 6 and in reference to a Cartesian orthogonal axes system, the perpendicular to the substrate is the axis Z, the plane of the substrate top surface is XY. In each structure of FIG 6, an element arising for the etching of a set of recess structures can be identified with an index i, the subsequent element is identified with index i+1, the previous element is identified with index i-1, with i=0 the i-element is placed in the center of the substrate, with i=±m the i-element is placed at the edge of the substrate, wherein m is a integer number higher than 1. The i-element forms a set of slant angles with respect to Z. In a first approximation, the set of slant angles of the i-element comprises at least the slant angle αi with respect to Z in the plane XZ and the slant angle βi with respect to Z in the plane YZ, wherein βi may or may not differ from αi, wherein αi and βi vary between a non-zero value for i=±m (edge) and zero for i=0 (center).

In one embodiment, a slanted circular or linear Fresnel zone-plate is fabricated with the process flow described as follows: a layer of Cr hard mask with a thickness of 20 to 50 nm is deposited on the surface of a thin silicon substrate (10 to 100 µm), and a layer of PMMA resist is then coated on the top of the hard mask. The circular or linear Fresnel zone-plate with an outermost zone width down to 5 nm is exposed using electron beam lithography with a typical element size ranging from 50 µm to a few mm. The as-developed PMMA resist is then pre-treated with oxygen plasma to remove any residual resist, and the Fresnel zone plate pattern is transferred into the Cr layer by plasma etching with chlorine and oxygen as etchant gases. The modulator matching the optical element in size and geometry is then glued or lithographically produced around the patterned Fresnel zone-plate. In case of a patterned area with a circular Fresnel zone plate, the electric field modulator is produced with a circular shape concentric and enclosing the patterned area. The size of circular electric field modulator surrounding the patterned area is simulated and selected on the basis of the desired slant angle. The thickness of the modulator is chosen to obtain the suitable slant angle. The silicon DRIE process is performed using the Cr hard mask. The fabrication of the slanted Fresnel zone-plate in Si substrate may be combined with a following step of electroplating or atomic layer deposition of materials with higher refractive index for designed X-ray energy such as Au, Ir, Ru, Pt, W, Ta, Pb, Bi, Sn, Cu, Ni.

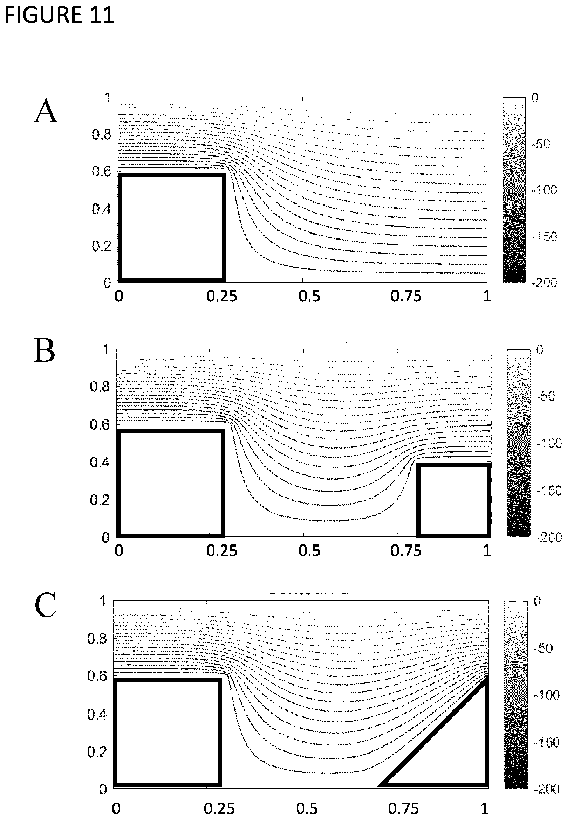

In some embodiments, an off-axis optical device is fabricated, which consist of only a fraction of Fresnel zone plate. In this case, the fan-shaped structure is not symmetric with respect to the center. The asymmetric fan-shaped structure is produced by using asymmetric modulators. The asymmetric modulators may consist of but not limited to: two or more modulators with different shape, two or more modulators with same shape but different size, two or more modulators with different shape and size. Some examples of asymmetric modulators are reported in FIG.11. In some embodiments the use of one modulator might be sufficient (FIG. 11A).

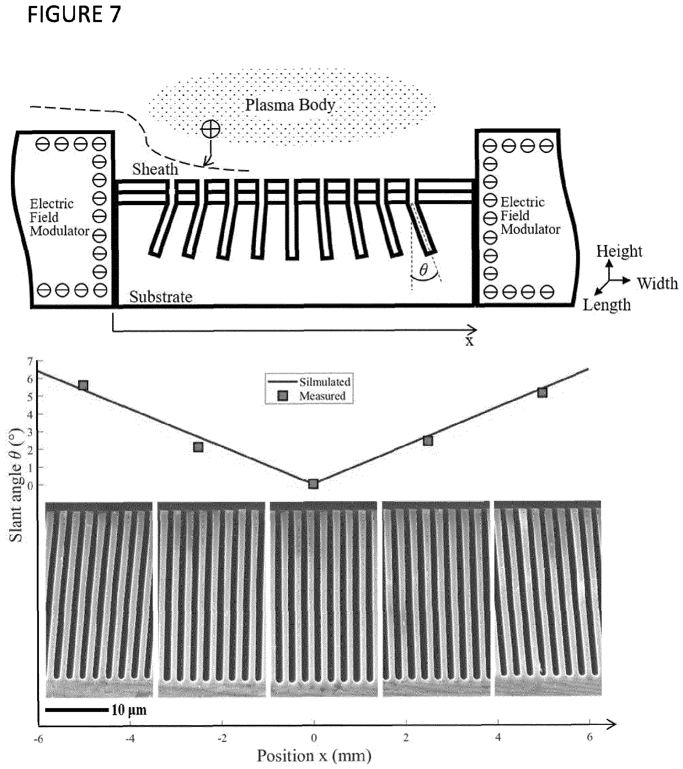

In one embodiment, a 1D fan-shaped grating is fabricated with the process flow described as follows: a layer of Cr hard mask with a thickness of 50 nm is deposited on the surface of a silicon wafer, and a layer of MICROPOSIT S1805 photoresist is then coated on top of the hard mask. The grating pattern with a period of 3 µm and duty cycle of 0.5 into the photoresist is exposed to UV light in a conventional mask aligner. The as-developed photoresist is then pre-treated with oxygen plasma to remove any residual polymer, and the grating pattern is transferred into the Cr layer by plasma etching with chlorine and oxygen as etchant gases. A chip is cleaved out of the aspatterned Si wafer, and serves as the substrate. The dimensions of the Si chip are 10 mm (width) × 30 mm (length) × 300 µm (thickness). A single side polished Si wafer is used as a carrier wafer. A silicon oxide layer is introduced to reduce the etchant consumption and to effectively stop the electrons from being transferred to the ground during the etching process. The substrate is placed in the central area of the carrier wafer, and is fixed with bonding wax. Two metal slabs are used as electric field modulators, and each metal slab has dimensions of 5 mm (width) × 30 mm (length) × 1 mm (thickness). The metal slabs are placed next to each side of the substrate, with long edges in parallel with the grating lines as shown in FIG 2 and FIG 7. The metal slabs are fixed with bonding wax. The bonding wax is used to fix the relative positions of the electric modulators and the patterned chip in order to improve the stability of the structure and the reproducibility of the slanting geometry, and to facilitate the handling and the positioning of the electric modulators and the patterned chip within the etching chamber. The method here disclosed can be performed also in presence of different bonding medium or even without bonding medium if an insulating layer is provided. In this example, the silicon oxide layer can be used as insulating layer. The DRIE process is performed with an Oxford PlasmaLabSystem100 ICP-type etcher. The pressure of the chamber is maintained at 25 mTorr throughout the entire process. The flow of sulfur hexafluoride gas is set at 120 sccm during the etching sub-steps and 20 sccm during deposition sub-steps. The flow of octafluorocyclobutane gas is set at 10 sccm during the etching sub-steps and 110 sccm during deposition sub-steps. The RF power is set at 50 W and ICP power is set at 800 W during the whole process. There are 6 seconds of etching and 7 seconds of deposition within each loop, and 150 loops are conducted in total.

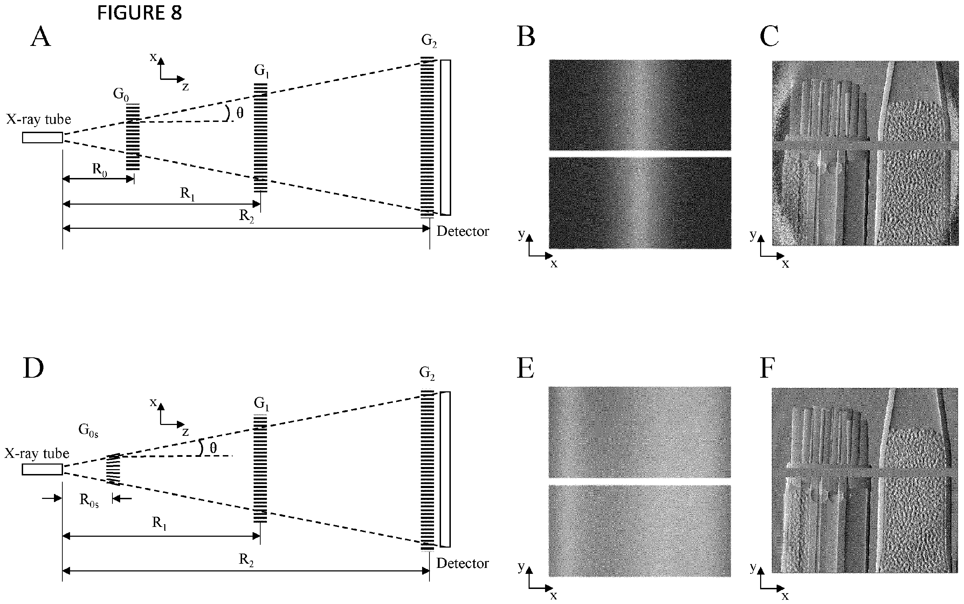

FIG 7 are SEM images in cross-section taken from the 1D fan-shaped grating in silicon substrate produced in one embodiment to this disclosure. The etched fan-shaped grating has been diced perpendicularly to the grating lines and SEM images are taken from different points along the slice moving from one edge to the other, and the distances between neighboring points are the same. The SEM images are representative to show the variation of slant angle moving from one edge to the other. The SEM images of FIG.7 were acquired in the same SEM session after etching approximately 25 µm, the SEM stage has a fixed tilt so the observed tilt is not a microscopy artifact but it related to the etching. The SEM image in the center to the grating shows the lines perpendicular to the substrate as expected for zero slant angle. The slant angle grows with a gradient and symmetrically in both directions from the center to edges ranging from 0° to 6.6°. The trenches were etched up to approximately 40 µm deep and then partially filled with Au by electroplating in a Au cyanide bath and used as absorption grating G₀ in a grating based X-ray imaging set-up of FIG 8.

FIG 8 schematically illustrates a grating-based X-ray imaging setup composed of an X-ray source, an absorption grating G₀, a sample, a phase grating G₁, an absorption analyzer grating G₂, and an X-ray detector. All the grating lines are oriented in Y direction according to Cartesian coordinates orientation marked near the images. The conventional set-up with regular gratings is showed in FIG 8A. FIG 8B shows the transmission image of the grating G₀. FIG 8C shows the differential phase contrast image from a real measurement of a sample composed of particles in a tube and some polymeric slabs obtained in a conventional set-up (FIG 8A) with the regular gratings. In reference to FIG 8D, the 1D fan-shaped grating produced following one embodiment of this disclosure is represented as G₀ₛ. The 1D fan-shaped grating is used as absorption grating G₀ₛ and positioned between the X-ray source and the phase grating G₁. FIG 8F shows the differential phase contrast image from a real measurement of a sample composed of polysterene particles in a tube and some polymeric slabs obtained in a set-up with a fan-shaped grating (FIG 8D).

Since the grating lines are slanted with a designed angle, the G₀ₛ grating can be placed closer to the X-ray source in comparison to the conventional set-up, meaning that the distance between the fan-shaped grating (R₀ₛ) can be much smaller than the conventional type of imaging set-up (R₀). Taking the magnification effect from the experimental setup into account, a fan-shaped G₀ₛ grating with much smaller size is required to cover the same field of vision (FOV), or to say a larger FOV can be covered with the same sized G₀/G₀ₛ grating.

The G₁ and G₂ gratings on both two types of setups are conventional gratings with gratings line perpendicular to the substrate and produced in Si substrates by DRIE (G₁) and DRIE followed by Au electroplating (G₂), respectively. The three gratings of the conventional set-up are fabricated in Si by DRIE, the absorption gratings G₀ and G₂ are filled with Au electroplating after DRIE. X-ray imaging comparing experiments are carried out with the two set-ups, the gratings G₁ and G₂ are the same in both set-ups. From the differential phase contrast images of the sample, a significant improvement in the signal to noise ratio of the setup is observed by replacing the conventional absorption grating (FIG 8C) with the fan-shaped absorption grating (FIG 8F), especially at the edge areas where the required slant angle becomes relatively larger.

Given a certain detector size (2d), a given distance between the X-ray source and the detector (R₂), a given distance between the X-ray source and the G₀ grating (R₀ₛ), the size and the slant angle of the G₀ grating are chosen so that the slant angle at the edge of the G₀ grating (θ) matches the angle of the X-ray ray hitting the edge of the detector (θ=arctan(d/R₂)). In this case, the FOV of the imaging system is optimal. An angle mismatch between the lines of G₀ and the rays of the X-ray source results in a decrease of the X-ray intensity at the edges of the X-ray detector in the direction perpendicular to the grating lines, as shown in FIG.8.

The variation of the slant angle as a function of the position of the slanted element in the fan-shaped recess structure is chosen as a function of the final application of the optical component. For example, focusing elements, such as Fresnel zone-plates may require a strong angle variation close to the edge of the pattern, while linear gratings need a soft gradual modulation of the slant angle from edge to the center of the pattern.

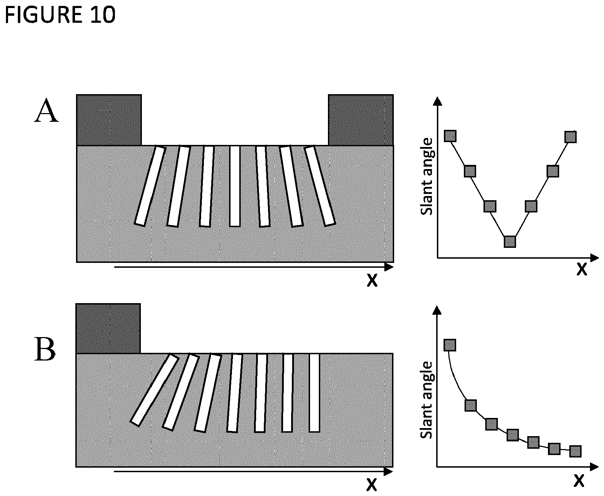

When the electric field modulator is placed only at one side of the pattern to be etched as shown in FIG. 10B, the slant angle is relatively large for the etched recess structures close to the electric field modulator, but the slant angle also drops faster as a function of the distance from the modulator. Moreover, the slant angle asymptotically reaches the zero value as a function of the distance from the modulator (FIG. 10B). With electric field modulators existing at both sides of the pattern to be etched (FIG. 10A), under the joint influence of the electric field modulators from both sides, the slant angle of the etched recess structure gradually decreases as a function of the distance from each modulator, and a vertical etching (zero slant angle) can be achieved at the center of the etching pattern if two symmetric modulators are applied. With asymmetric design, the vertical etching area (zero slant angle) is shifted towards the electric field modulator that creates weaker electric field, and the maximum slant angle is smaller near the weaker side. Therefore, the presence of a second modulator (FIG. 11) offers a better option to control the slant angle variation as a function of the distance from the modulator. In some embodiments of this disclosure, two or more modulators are used to create an asymmetric slant angle variation with respect to the center of the pattern.

In some embodiments of this disclosure, an asymmetric variation of slant angle in the fan-shaped structure with respect to the center of the pattern is realized (FIG. 10). The electric field modulators might be not symmetric in shape or size (FIG. 11) so that the produced fan-shaped structure has slanted elements in the substrate with a non-symmetric distribution of slant angle with respect to the center of the pattern (FIG. 10B).
In one embodiment of this disclosure, a 2D fan-shaped grating with slant angle in the fan-shaped structure with respect to the center of the pattern is realized (FIG. 12) by means of an electric modulator made of a metallic ring, wherein the metallic ring is surrounding the patterned chip, wherein the patterned chip has a grating array of a chessboard. The SEM images in different positions (FIG.12 B) of the etched chip have been acquired in plan view, all images have been acquired in the same SEM session without modifying the relative tilt angle of SEM stage so the observed tilt is not a microscopy artifact but it is related to the etching. The SEM images in different positions (FIG.12 B) of the etched chip show the increase of slanting angle of the silicon recess structures as a function of the distance from the ring center. Each silicon recess structure is inclined in planes perpendicular to the surface of the substrate, wherein the recess direction forms a slant angle with the perpendicular to the surface of the substrate, wherein the said slant angle gradually varies from one recess to the next recess as a function of the recess location on the substrate.

### List of Reference Numerals

- 100: Carrier wafer
- 200: Insulating bonding material
- 301: electric field modulator
- 302: electric field modulator
- 400: substrate
- 401-409: trenches etched into substrate 400
- 450: seed layer for electroplating
- 460: carrier substrate
- 501-503: equipotential lines in etching chamber
- 601-609: ion acceleration directions
- 700-720: photoresist layer with grating pattern
- 800: hard mask with grating pattern

## Claims

1. A method for fabricating an optical component having high aspect ratio elements (401 to 409), such as recess structures, with a fan-shape orientation in a substrate (400), wherein each element (401 to 409) is inclined in either one or more freely selectable directions in planes perpendicular to the surface of the substrate (400), wherein the element direction forms a slant angle with the perpendicular to the surface of the substrate (400), wherein the said slant angle gradually varies from one element to the next element as a function of the element location on the substrate (400), comprising the steps of:
a) providing a substrate (400) having a pattern of the optical component on the upper surface, wherein said pattern serves as a mask (800) for a following etching step;
b) placing one or more electric field modulators (301, 302) next to the patterned surface of the substrate (400);
c) inserting the patterned substrate (400) with the electric field modulators (301, 302) into a chamber of a plasma etching system, wherein the electric field modulators (301, 302) are insulated from an etcher cathode in the etching chamber;
d) forming the high aspect ratio recess structures (401 to 409) in the patterned substrate (400) by means of etching with accelerated ions in the modified electric field formed by the presence of the electric field modulators (301, 302) located next to the patterned surface of the substrate (400).

2. The method according to claim 1, wherein the substrate (400) is a single or a multilayer, wherein the material of each layer is selected from the group consisting of: Si, SiO₂, Al₂O₃, Ge, an alloy of Si and Ge, SiN, SiC, diamond, graphite, Mo, W, C, Ta, glass, polymer, and combination thereof.

3. The method according to claim 1, wherein the substrate (400) is a single or a multilayer, wherein the material of each layer is selected from the group consisting of: elements and alloys from the groups III and V of the periodic table, elements and alloys from the groups II and VI of the periodic table, and combination thereof.

4. The method according to claim 2, wherein the material of the substrate (400) is heavily doped silicon, wherein the silicon dopants are selected from the group consisting of: Gd, B, Cd, Co, Hf, Ti, Mo or combination thereof.

5. The method according to any of the preceding claims, wherein the pattern is realized in a photoresist, electron beam resist, or imprint resist layer by a lithographic method selected from the group consisting of: UV mask lithography, displacement Talbot lithography, electron-beam lithography, imprint lithography and combination thereof.

6. The method according to any of the preceding claims, further comprising two optional steps of:
a) depositing at least a layer of material beneath the photoresist layer as hard mask (800) for the etching step;
b) transferring the pattern into the hard mask layer by etching after the lithographic step.

7. The method according to claim 6, wherein the hard mask (800) is selected from the group consisting of: Al, Ti, Cr, Al₂O₃, SiO₂ and combination thereof.

8. The method according to any of the preceding claims, wherein:
a) the said patterned substrate (400) is placed onto a carrier wafer (100);
b) the electric field modulators (301, 302) are placed next to the substrate (400) onto the carrier wafer (100);
c) the patterned substrate (400) and the electric field modulators (301, 302) are fixed by means of an insulating adhesive material;
d) wherein the carrier wafer (100) together with the fixed substrate (400) and the fixed electric field modulators (301, 302) are placed into the chamber of the etching plasma;
e) wherein the said etching is carried out by means of reactive ion etching.

9. The method according to any of the preceding claims, wherein:
a) the said patterned substrate (400) is placed onto a carrier wafer (100);
b) the electric field modulators (301, 302) are placed next to the substrate (400) onto the carrier wafer (100);
c) the patterned substrate (400) and the electric field modulators (301, 302) are separated by the carrier wafer with a layer of an insulating material;
d) wherein the carrier wafer (100) together with the substrate (400) and the electric field modulators (301, 302) are placed into the chamber of the etching plasma;
e) wherein the said etching is carried out by means of reactive ion etching.

10. The method according to claims 8 or 9, wherein the patterned substrate (400) is placed onto a carrier wafer (100) and the electric field modulators (301, 302) are placed on top of the substrate (400) and next to the patterned area.

11. The method according to claim 8, wherein the insulating adhesive material is selected from the group consisting of:
bonding wax, epoxy resin, polyimide films, and combination thereof.

12. The method according to any of the preceding claims, wherein the electric field modulators (301, 302) are made from conductive materials selected from the group consisting of: Al, Au, W, Ag, Pt, Cu, Fe, Ni, steel, graphite, and combination thereof.

13. The method according to any of the preceding claims, wherein the electric field modulators (301, 302) having a shape selected from the group consisting of slab, annulus, sheet, foil, layer and combination thereof, wherein:
a) the slab having a cross sectional shape selected from the group consisting of: rectangle, triangle, trapezoid, semicircular and combination thereof;
b) the annulus having a cross sectional shape selected from the group consisting of: rectangle, triangle, trapezoid, semicircle and combination thereof;
c) the sheet, foil and/or layer having an open area, wherein the shape of the said open area encloses the patterned area of the substrate (400).

14. The method according to claim 10, 12 or 13, wherein the fabrication of electric modulators is selected from the group consisting of: evaporation, etching, lithography, deposition assisted by scanning focused ion beam and scanning electron beam, hot embossing, casting, and combination thereof.

15. The method according to any of the preceding claims, wherein reactive ion etching is carried out with essential parameters of the process comprising:
a) a pressure of the plasma etching chamber within the range from 1 mTorr to 100 mTorr during the process;
b) a cathode temperature of the plasma etching chamber is maintained within the range from -150°C to 30°C;
c) a process gas is selected from the group consisting of: fluorine based gases, chlorine based gases and oxygen, and combination thereof, wherein the gas flows are within the range from 0 to 1000 sccm;
d) a power supply comprising of upper source ICP power and bottom platen RF power, wherein the ICP power is within the range from 0 to 5000 W and the RF power is within the range from 0 to 1000 W.

16. The method according to any of the preceding claims , further comprising a partial or complete filling of the volume of the etched recess structures (401 to 409) with a filling material that is different from the material of the substrate (400), wherein the filling process is selected from the group consisting of: evaporation, laser ablation, chemical vapor deposition, atomic layer deposition, sputtering, epitaxy, electroplating, electro-less plating, crystal growth from a seed layer, casting from melted alloys, casting from solutions of dispersed particles, powder compression, particles precipitation from solvent evaporation, sol-gel synthesis, deposition assisted by scanning focused ion beam and scanning electron beam, and combination thereof.

17. The method according to claim 16, wherein the filling material is selected from the group consisting of: Au, Ir, Pt, Ru, Os, W, Gd, Ta, Pb, Bi, Sn, Cu, Ni and combination thereof.

18. The method according to claim 16, wherein the filling material is a scintillating material selected from the group consisting of: Gd₃Ga₅O₁₂:Tb, CdWO₄, Y₃Al₅O₁₂:Ce and combination thereof.

19. An apparatus for grating based X-ray imaging, comprising:
a) an X-ray source (x-ray tube);
b) an X-ray sensitive detector (Detector);
c) a set of three gratings (G₀ₛ, G₁, G₂) positioned between the X-ray source and the detector;
d) at least one of the gratings having fan-shape structure in a substrate (400) and being manufactured according any of the claims 1 to 18;
e) at least one of the said gratings having fan-shape structure has the volume of the etched recess structures (401 to 409) at least partially filled with a material selected from the group consisting of Au, Ir, Pt, Ru, Os, W, Gd, Ta, Pb, Bi, Sn, Cu, Ni and combination thereof;
f) at least one of the filled fan-shaped grating is positioned between the X-ray source and one of the gratings acting as phase grating (G₁); and
g) the maximum slant angle of the said fan-shaped grating is 45°.
